# EUROPÄISCHE PATENTSCHRIFT

(11) **EP 0 951 920 B1**
(45) Veröffentlichungstag und Bekanntmachung des Hinweises auf die Patenterteilung: **20.10.2004**
(21) Anmeldenummer: 99250128.8
(22) Anmeldetag: 21.04.1999
(51) Int. Cl.: A61N 1/05

(54) **Gefässelektrodenleitung**
Electrode cable for attachement to a vessel wall
Electrode destinée à être fixée à la paroi d'un vaisseau

(30) Priorität: 22.04.1998 DE 19818908; 18.08.1998 DE 19838360
(43) Veröffentlichungstag der Anmeldung: 27.10.1999
(73) Patentinhaber: BIOTRONIK Mess- und Therapiegeräte GmbH & Co Ingenieurbüro Berlin, 12359 Berlin (DE)
(72) Erfinder: Witte, Joachim, 10409 Berlin (DE); Flach, Erhard, 12305 Berlin (DE); Rexhausen, Hermann, verstorben (DE)
(74) Vertreter: Eisenführ, Speiser & Partner

(56) Entgegenhaltungen:
- EP-A- 0 601 338
- EP-A- 0 601 340
- EP-A- 0 606 688
- US-A- 4 332 259
- US-A- 4 550 737
- US-A- 5 405 374
- US-A- 5 476 498

## Beschreibung

Die Erfindung betrifft eine Gefäßelektrodenleitung für einen Herzschrittmacher.

Bei der Therapie verschiedener chronischer Herzrhythmusstörungen sind seit langem implantierte Herzschrittmacher in Verbindung mit auf einem intrakardialen Elektrodenkatheter angeordneten und an der Herzinnenwand positionierten Stimulationselektroden in Gebrauch, über die das reizbare Herzgewebe erregt und dadurch ein Defekt des körpereigenen kardialen Reizbildungs- und -leitungssystems ausgeglichen wird.

Die Ausführung der Elektrodenleitungen ist immer weiter vervollkommnet worden. Mit dem Ziel einer langfristigen Gewährleistung eines guten Kontaktes zwischen der oder den Stimulationselektrode(n) und dem Herzgewebe im Interesse einer zugleich energiesparenden und sicheren Stimulation sind zahlreiche technische Lösungen zur Verankerung der Elektrodenleitungen an der Herzwand - sowohl in der Herzkammer (Ventrikel) als auch im Vorhof (Atrium) - gefunden worden und tatsächlich wesentliche praktische Verbesserungen gelungen.

Es sind auch Elektrodenleitungen - speziell für implantierbare Defibrillatoren - vorgeschlagen worden, die in große herznahe Gefäße eingeführt werden und die Defibrillationsenergie an die Gefäßwand abgeben.

Die EP 0 601 338 A1 beschreibt ein solches Elektrodensystem für einen implantierten Defibrillator, das mindestens zwei - ohne besondere Verankerung allein aufgrund ihrer Größe ortsfest gehaltene - intravaskular plazierte Spulenelektroden (spiralförmige Elektroden) umfaßt. Eine dieser großflächigen Defibrillationselektroden kann speziell in der Vena cava superior angeordnet sein, eine weitere im Koronarsinus.

Die US 5,571,159 beschreibt einen temporär einsetzbaren Katheter zur atrialen Defibrillation, der neben einem ersten, im Atrium positionierten spiraligen Elektrodenabschnitt einen zweiten, in der Pulmonararterie plazierten Elektrodenabschnitt und am distalen Ende einen aufblasbaren Ballon zur Positionierung der Elektroden aufweist.

Die EP 0 566 652 B2 beschreibt eine in Art eines Stents geformte und mittels eines Dilatations-Ballons aufweitbare Elektrodenkonfiguration zum Einsatz als Defibrillationselektrode in einem Blutgefäß, speziell im Koronarsinus.

Für bestimmte Anwendungen - etwa die sogenannte biatriale oder Multisite-Stimulation - kann auch eine Plazierung von Schrittmacher-Stimulations- oder Abfühlelektroden in einem herznahen Gefäß, speziell im Koronarsinus, zweckmäßig sein, weil eine Elektrodenimplantation speziell im linken Atrium problematisch ist. Hierfür eignen sich die bekannten großflächigen Defibrillationselektroden grundsätzlich nicht.

Die EP-A-0 606 688 beschreibt eine Gefäßelektrodenleitung gemäß dem Oberbegriff von Anspruch 1.

Der Erfindung liegt daher die Aufgabe zugrunde, daß in der Praxis der für eine Fixierung der Leitung am besten geeignete Ort nicht in fester Positionsbeziehung zu dem für die Stimulation am besten geeigneten Ort des Gefäßes steht.

Diese Aufgabe wird durch eine Gefäßelektrodenleitung mit den Merkmalen des Anspruchs 1 gelöst.

Der Erfindung liegt die Überlegung zugrunde, den elektrisch aktiven Teil der Anordnung gegenüber dem zur mechanischen Fixierung vorgesehenen Teil in gewissen Grenzen frei positionierbar zu machen. Dies ist in zweckmäßiger Weise durch das Vorsehen separater Träger für die Elektroden einerseits und das oder die Fixierungsmittel andererseits zu realisieren, die während der Plazierung der Elektrodenleitung relativ zueinander bewegbar sind und deren Stellung nach Ermittlung der optimalen Position für das Fixierungsmittel und die Elektroden festgelegt wird. Die erfindungsgemäße Gefäßelektrodenleitung weist daher die kennzeichnenden Merkmale auf.

Die Erfindung schließt den Gedanken ein, eine Gefäßelektrodenleitung mit mindestens einer kleinflächigen Elektrode und an die Gefäßkonfiguration angepaßten Fixierungsmitteln zu realisieren, mit der in effizienter Weise und mit exakter

Positionierung Stimulationsimpulse auf die Gefäßwandung übertragen bzw. Herzaktionspotentiale abgegriffen werden können.

Zur Ausführung dieses Gedankens umfaßt die vorgeschlagene Elektrodenleitung eine Elektrode bzw. Elektroden, deren effektive Durchmesser (wie von intrakardialen Elektrdoenanordnungen bekannt) annähernd gleich dem Durchmesser des Grundkörpers ist und nicht als Elektrode wirkende (insbesondere eine isolierende Oberfläche aufweisende) Fixierungsmittel zur ortsfesten Positionierung der Elektrode bzw. Elektroden bezüglich der Gefäßwandung.

Die Fixierungsmittel der vorgeschlagenen Gefäßelektrodenleitung unterscheiden sich von denen intrakardial zu verankernder Elektrodenleitungen in der Gestalt, zumindest aber den Abmessungen, da sie nicht einer Fixierung durch Verankerung in relativ dickem Muskelgewebe oder dem Trabekelwerk des Ventrikels dienen. Vielmehr beruht die Fixierung entweder auf einer elastischen Verspannung gegen die Gefäßwand oder alternativ auf dem Eingriff in die Wandung bzw. Verzweigungen eines Nebengefäßes.

Erfindungsgemäß sind am distalen Ende des Grundkörpers flexible Fixierungsmittel zur Fixierung im besagten Seitengefäß (speziell des Koronarsinus) mit auf dessen Durchmesser abgestimmter lateraler Erstreckung angebracht, und der Grundkörper oder der Fixierungsmittel-Trägerkörper ist in diesem Bereich hochgradig biegsam ausgeführt, um ein leichtes Abwinkeln in das Seitengefäß hinein zu ermöglichen.

Als derartiges Fixierungsmittel ist bevorzugt ein das distale Ende des Fixierungsmittel-Trägerkörpers umgebender Kunststoff-Schneckengewindeabschnitt mit einer oder mehreren Windungen, insbesondere aus dem Material des Grundkörpers, vorgesehen, mit dem die Elektrodenleitung in einem kleineren venösen Seitengefäß des Koronarsinus aktiv fixiert werden kann. Dieses wird durch das Einschrauben " der Elektrodenleitung zwar weitgehend verschlossen, die damit bewirkte Stauung ist bei einem Seitengefäß aber tolerierbar.

Alternativ zur letztgenannten Ausführung kann der Grundkörper eine Mehrzahl von gegenüber seiner Längsachse zum proximalen Ende hin federnd geneigten Kunststoff-Finnen tragen, mit denen die Gefäßelektrodenleitung in Verzweigungen des herznahen Gefäßsystems passiv verankert werden kann. Die Abmessungen der Finnen bzw. "Flossen" sind in Abstimmung auf die andere Körperumgebung größer als bei ähnlichen Anordnungen, die im Trabekelwerk des Ventrikels verankert werden.

Weiterhin stellen eine während der Einführung streckbare elastische Wendel oder ein in Art eines Stents nach der Einführung aufweitbarer rohr- oder ringförmiger Hohlkörper mit an den Gefäßdurchmesser angepaßten Endabmessungen geeignete Fixierungsmittel dar.

Hierbei ist es von Vorteil, wenn die elektrisch aktive Oberfläche relativ klein bleibt. Das kann entweder durch mindestens teilweise Isolierung der Oberfläche oder mindestens teilweise Ausführung aus einem nichtleitenden Material oder auch durch minimale Dimensionierung des gesamten Fixierungselementes erreicht werden. Die Elektroden können so auf der Leitung plaziert werden, daß (bei einer unipolaren Leitung) die Elektrode oder (bei einer bi- oder multipolaren Leitung) die distalste von mehreren Elektroden einen Abstand zwischen 30 und 80 mm vom distalen Ende des Grundkörpers hat, so daß in Verbindung mit einem auf diesen Abstand abgestimmten Krümmungsverlauf des Grundkörpers im implantierten Zustand eine stabile Ausrichtung der Elektrode(n) relativ zur Gefäßwand gewährleistet ist. Entsprechend beträgt bei einer bipolaren Leitung der Abstand der Elektroden zwischen 30 und 80 mm. Auch der Einsatz einer Spitzenelektrode ist - allein oder in Kombination mit einer Ringelektrode - möglich.

Die erste und/oder zweite Stimulationselektrode weist bevorzugt eine geometrische Oberfläche im Bereich zwischen 10 und ca. 25 mm² und insbesondere eine fraktale Oberflächen-Mikrostruktur zur Vergrößerung der elektrisch wirksamen Oberfläche um einen Faktor von mindestens 10² auf.

Weiterhin ist bevorzugt eine das Einwachsen positiv beeinflussende Wirkstoffbeschichtung vorgesehen, etwa eine Kollagen- oder Fibronectin-Beschichtung.

Das Festlegen kann beispielsweise durch Crimpen oder durch eine spannzangenähnliche Einrichtung im Bereich des Steckers geschehen.

Vorteilhafte Weiterbildungen der Erfindung sind im übrigen in den Unteransprüchen gekennzeichnet . Es zeigen:
- Figur 1 eine schematische Darstellung einer Gefäßelektrodenleitung,
- Figur 2 eine schematische Darstellung einer weiteren Gefäßelektrodenleitung,
- Figuren 3, 3a eine schematische Darstellung eine weiteren Gefäßelektrodenleitung,
- Figuren 4, 4a eine schematische Darstellung einer weiteren Gefäßelektrodenleitung,
- Figur 5 eine schematische Darstellung einer weiteren Gefäßelektrodenteitung und
- Figuren 6a bis 6d schematische Darstellungen verschiedener Ausführungen eines Details bei den Gefäßelektrodenleitungen der in Fig. 3 bis 5 gezeigten Art.

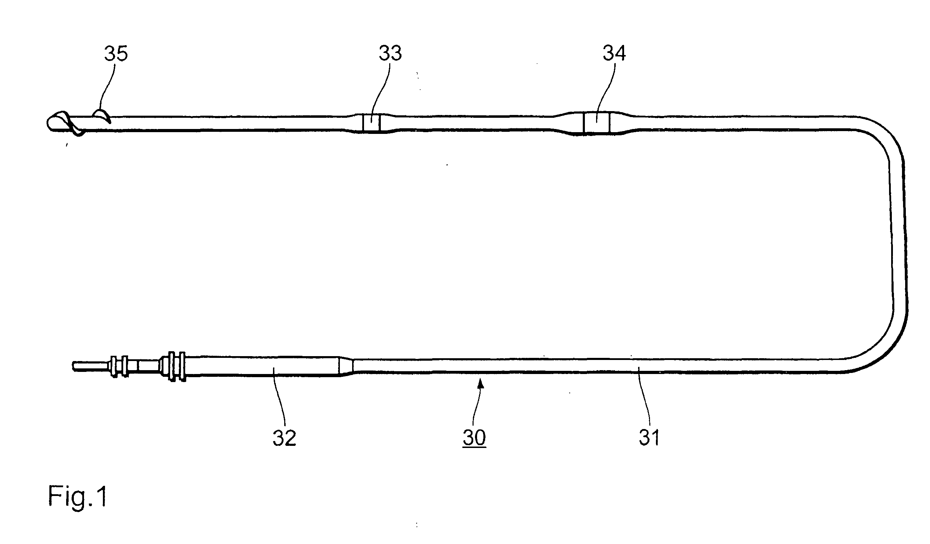

Eine im distalen Endabschnitt gestreckte Gefäßelektrodenleitung 30 mit einem Grundkörpers 31 und einem Stecker 32 ist in Fig. 1 dargestellt. Die Leitung 30 weist zwei Ringelektroden 33 und 34 mit Durchmessern von 2 bzw. 2,4 mm und Längen von 2 bzw. 3 mm auf. Die näher zum distalen Leitungsende gelegene Elektrode 43 hat zum Ende einen Abstand von 75 mm, und der Abstand zwischen beiden Elektroden beträgt 25 mm.

Am distalen Ende ist an den Grundkörper 31 eine aus einem Silikonpolymeren gebildete Schneckenwendel 35 von ca. 5 mm Länge mit zwei Windungen angefügt, die den Gesamtdurchmesser der Leitung in diesem Bereich auf ca. 3 mm vergrößert. Die Leitung wird bei der Implantation so geführt, daß das "Gewinde" an der Spitze in die Einmündung eines Seitengefäßes gelangt, und anschließend wird sie dort vermittels entsprechender Drehung des Führungsdrahtes eingeschraubt. Aufgrund der ausgeprägten Biegungselastizität der Leitung verspannt sich deren Endbereich nach der Fixierung bogenförmig von der Gefäßmündung des Seitengefäßes aus zur gegenüberliegenden Wandung des Hauptgefäßes (z.B. Koronarsinus), so daß die Elektroden mit dessen Wandung in Kontakt stehen, ohne daß ein zu großer lokaler Druck auf die Gefäßwand ausgeübt wird.

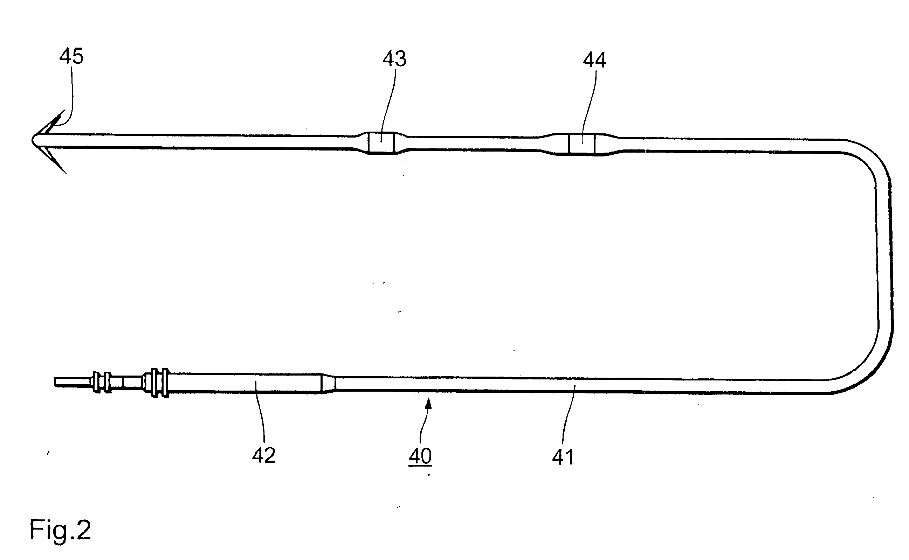

Eine weitere Gefäßelektrodenleitung 40 mit vom Stecker 42 bis zum distalen Ende durchgehend gestrecktem Verlauf des Grundkörpers 41 ist in Fig. 2 dargestellt. Die Leitung 30 weist zwei Ringelektroden 43 und 44 mit Durchmessern von 2 bzw. 2,4 mm und einer Länge von jeweils 3 mm auf. Die näher zum distalen Leitungsende gelegene Elektrode 43 hat zum Ende einen Abstand von 50 bis 60 mm, und der Abstand zwischen beiden Elektroden beträgt 30 bis 40 mm.

Am distalen Ende sind an den Grundkörper Kunststoff-Finnen bzw. -Widerhaken 45 (die bevorzugt aus dem Material des Grundkörpers, d.h. in der Regel einem Silikonpolymeren, gebildet sind) angeformt. Die Finnen 45 sind ausgeprägt spitzwinklig und länger als ähnliche Verankerungselemente von intrakardialen Elektrodenleitungen, jedoch biegsam ausgeführt. Die Leitung wird mittels des Führungsdrahtes so geführt, daß die Finnen sich in einer Gefäßverzweigung oder der Einmündung eines Seitengefäßes verhaken und so die Elektroden in geeigneter Lage relativ zur Wandung des für die Anbringung vorgesehenen Gefäßes, speziell des Koronarsinus, fixieren.

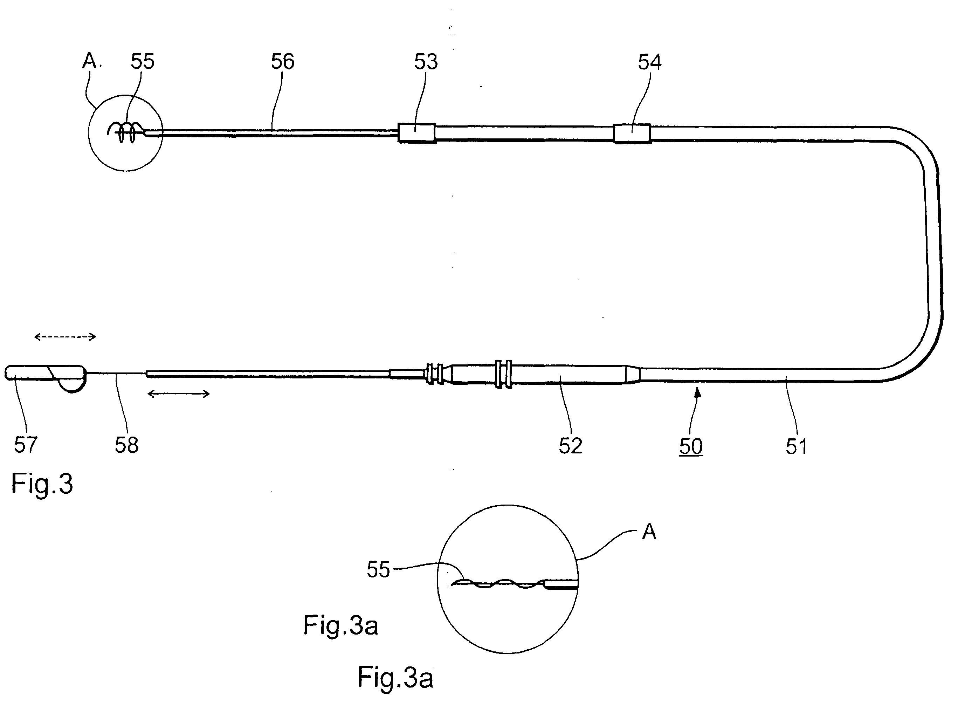

Fig. 3 und 3a zeigen schematisch eine weitere Gefäßelektrodenleitung 50.

Hinsichtlich der Anordnung des Grundkörpers 51, des Steckers 52 und der Elektroden 53, 54 gleicht diese den Anordnungen nach Fig. 1 oder 2 so daß insoweit die Beschreibung nicht wiederholt wird. Jedoch ist hier der Grundkörper 51 so ausgeführt, daß er in axialverschieblicher Weise einen gegenüber dem Grundkörper um einige Zentimeter längeren Fixierungselement-Trägerkörper 56 aufnimmt. An dessen distalem Ende ist eine hochelastische Wendel 55 aus biokompatiblem Metall (etwa einer NiTi-Legierung) oder Kunststoff befestigt. Auch der Trägerkörper 56 ist hohl, so daß in ihn ein Führungsdraht 58 einführbar ist, an dessen proximalem Ende ein zweiteiliger Handgriff 57 angebracht ist.

Der Führungsdraht 58 greift in ein entsprechend ausgebildetes Ende der elastischen Wendel 55 ein, so daß diese durch Vorschieben des Führungsdrahtes 58 gegenüber dem Fixierungselement-Trägerkörper 56 gestreckt werden kann, wie in der Darstellung des Ausschnitts A aus Fig. 3 in Fig 3a zu erkennen ist.

Dies erlaubt ein leichtes Einführen der Leitung 50 mit gestreckter Wendel 55. Nach Erreichen des gewünschten Plazierungsortes wird beim Zurückziehen des Führungsdrahtes die Wendel entlastet und nimmt ihre vorgeprägte Form an, in der sie einen derart an die Innenabmwessungen des Gefäßes angepaßten Durchmesser hat, daß sie an dessen Wandung unter leichtem Druck anliegt.

Durch Axialverschiebung des Grundkörpers 51 auf dem somit fixierten Fixierungselement-Trägerkörper 56 mittels des zweiten Teils des Handgriffs 57 kann anschließend die optimale Positionierung der Elektroden 53, 54 im einem relativ großen Einstellbereich vorgenommen werden. Nach erfolgter Positionierung wird schließlich (auf weiter unten genauer beschriebene Weise) die Stellung des Grundkörpers relativ zum Fixierungselement-Trägerkörper festgelegt und das über den Steckerstift 52 ggfs. noch überstehende proximale Ende des Trägerkörpers 56 abgetrennt, womit die Implantation der Leitung 50 beendet ist.

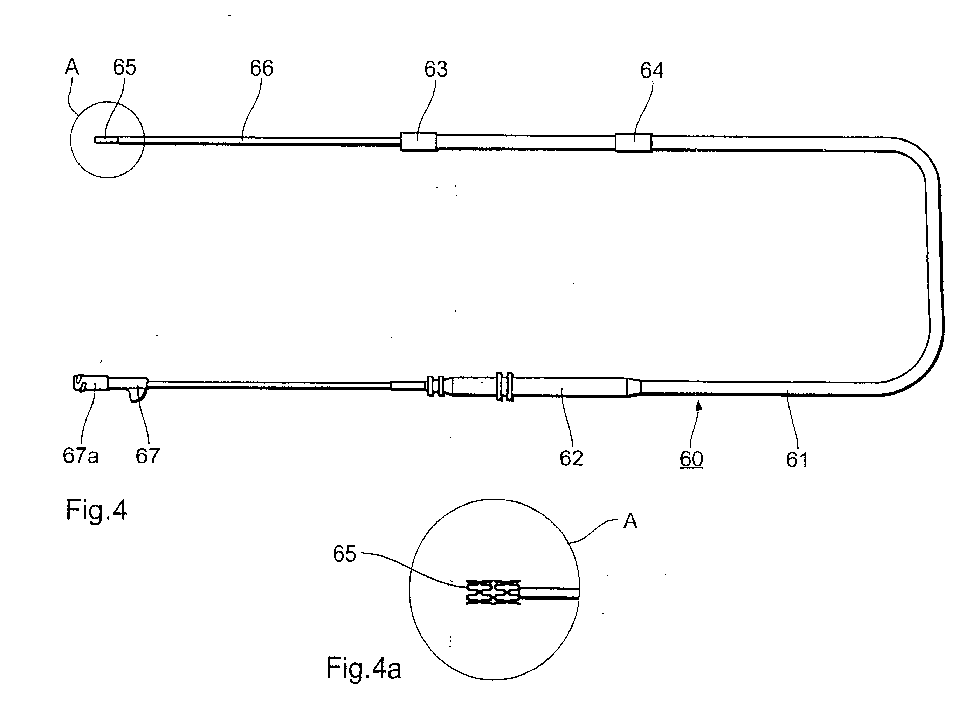

Fig. 4 und 4a zeigen eine in Aufbau und Funktion der oben beschriebenen Leitung nach Fig. 3 ähnliche Gefäßelektrodenleitung 60, bei der aber anstelle einer elastischen Wendel ein nach der Einführung in Art eines Stents aufweitbares rohrförmiges Fixierungselement 65 aus einer Ti-Legierung vorgesehen ist. Der Handgriff 67 dient hier nur zur Verschiebung des Fixierungselement-Trägerkörpers 66 relativ zum Grundkörper 61. Er weist zusätzlich einen Luer-Lock-Anschluß 67a zur Verbindung mit einer (nicht gezeigten) Fluidquelle auf, die zur Aufweitung des Fixierungselementes 65 durch Ballon-Dilatation in an sich bekannter Weise benötigt wird. Den aufgeweiteten Zustand zeigt die Darstellung des Ausschnitts A in Fig. 4a.

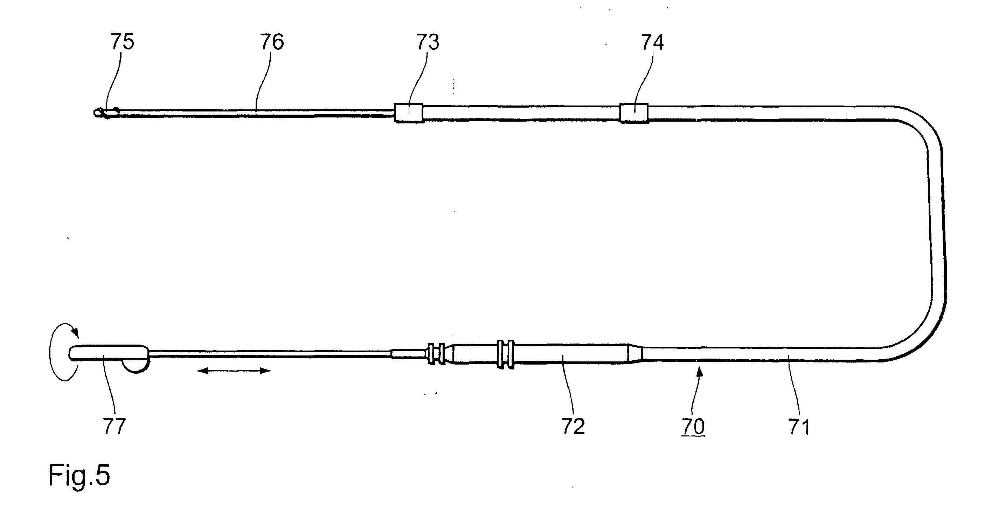

Fig. 5 zeigt eine der Ausführung nach Fig. 1 ähnliche Gefäßelektrodenleitung 70, bei der aber - wie bei den Anordnungen gemäß Fig. 3 und 4 für die schneckenartige Einschraubwendel 75 ein im Leitungs-Grundkörper 71 aufgenommener und diesem gegenüber axial verschieblicher Trägerkörper 76 vorgesehen ist, an dessen proximalem Ende ein Handgriff 77 zur Verschiebung des Trägerkörpers 76 und zum Einschrauben der Schneckenwendel 76 angebracht ist.

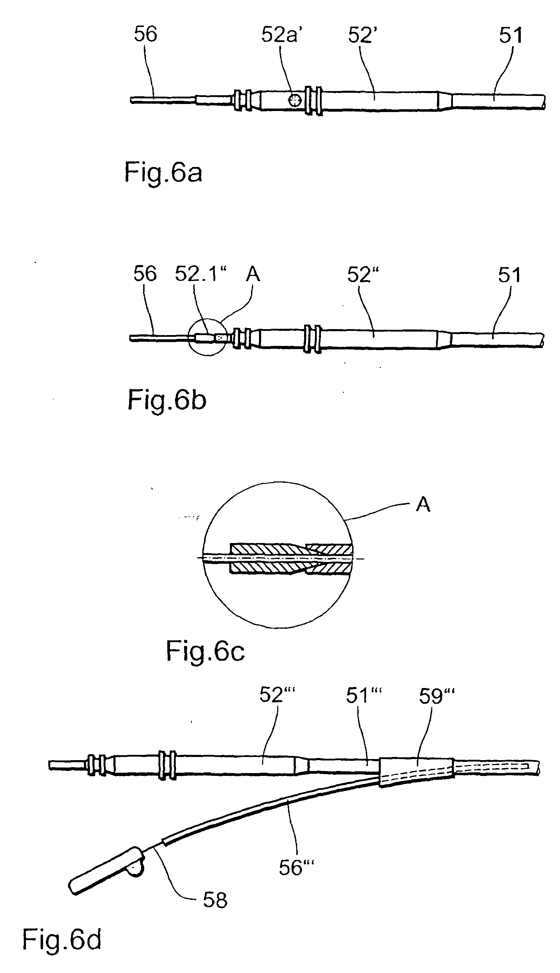

Fig. 6a bis 6d zeigen in Prinzipskizzen verschiedene Ausführungen eines wesentlichen Details bei Gefäßelektrodenleitungen der in Fig. 3 bis 5 gezeigten Art, nämlich der Mittel zum Festlegen der Stellung des Fixierungsmittel-Trägerkörpers relativ zum Grundkörper. In Fig. 6a ist zu diesem Zweck im Stecker 52' eine auf den Fixierungsmittel-Trägerkörper 56 angreifende Madenschraube 52a vorgesehen, in Fig. 6b ist der innere Steckerstift 52.1" eines modifizierten Steckers 52" aus zwei in Art einer Spannzange zusammenwirkenden Teilen 52.1 a" und 52.1b" gebildet, wie am besten in der Querschnittsdarstellung des Ausschnitts A aus Fig. 6b in Fig. 6c zu erkennen ist.

Bei der Ausführung nach Fig. 6d schließlich ist der Fixierungselement-Trägerkörper 52"' distal vom Stecker 52"' mit einem von verzweigten Elektrodenleitungen an sich bekannten - Abzweigelement 59"' versehen, in dem der Fixierungsmittel-Trägerkörper 56"' (und mit diesem der Führungsdraht 58) seitlich in den Grundkörper 51"' eingefädelt ist. Das Festlegen nach erfolgter Positionierung erfolgt hier mittels einer Ligatur.

## Patentansprüche

1. Gefäßelektrodenleitung (50, 60, 70) zur Schrittmacherstimulation und/oder zum Abfühlen von Herzaktionen im Bereich des Atriums, mit einem langgestreckten, mindestens eine Elektrodenleitung aufnehmenden Grundkörper (51, 61, 71) und mindestens einer am Grundkörper angebrachten Elektrode (53, 54, 63, 64, 73, 74), wobei der effektive Durchmesser der Elektrode bzw. Elektroden (53, 54, 63, 64, 73, 74) annähernd gleich dem Durchmesser des Grundkörpers (51, 61, 71) ist und im wesentlichen nicht als Elektrode wirkende, insbesondere mindestens teilweise eine isolierende Oberfläche aufweisende, Fixierungsmittel (55, 65, 75) zur ortsfesten Positionierung der Elektrode bzw. der Elektroden (53, 54, 63, 64, 73, 74) bezüglich der Gefäßwandung vorhanden sind, **dadurch gekennzeichnet, dass** im distalen Endbereich des Grundkörpers (51, 61, 71) ein an einem gegenüber dem Grundkörper (51, 61, 71) axial verschieblichen und in seiner axialen Stellung an diesem festlegbaren Fixierungsmittel-Trägerkörper (56, 66, 76) angebrachtes Fixierungsmittel (55, 65, 75) zur Fixierung im Koronarsinus oder in einem Nebengefäß desselben mit auf den Durchmesser des Gefäßes abgestimmter lateraler Erstreckung, insbesondere 3 bis 6 mm, vorgesehen ist und der Grundkörper oder der Fixierungsmittel-Trägerkörper (56, 66, 76) in diesem Bereich hochgradig biegsam ist.

2. Gefäßelektrodenleitung nach Anspruch 1, **dadurch gekennzeichnet, daß** als Fixierungsmittel ein das distale Ende des Fixierungsmittel-Trägerkörpers (71) umgebender Kunststoff-Schneckengewindeabschnitt (35; 75), insbesondere aus dem Material des Grundkörpers (31) oder des Fixierungsmittel-Trägerkörpers (71) und mit einer bis drei Windungen, vorgesehen ist.

3. Gefäßelektrodenleitung nach Anspruch 1, **dadurch gekennzeichnet, daß** als Fixierungsmittel eine Mehrzahl von gegenüber der Längsachse des Grundkörpers (41) zum proximalen Ende hin federnd geneigten Kunststoff-Finnen (45) vorgesehen ist.

4. Gefäßelektrodenleitung nach Anspruch 1, **dadurch gekennzeichnet, daß** als Fixierungsmittel eine elastische Wendel (55) vorgesehen ist, deren Durchmesser während der Einführung in einem durch ein Einführungshilfsmittel (58) gestreckten Zustand höchstens gleich dem Durchmesser des Grundkörpers und im entspannten Zustand größer als dieser ist.

5. Gefäßelektrodenleitung nach Anspruch 1, **dadurch gekennzeichnet, daß** als Fixierungsmittel ein aufweitbares rohrförmiges Element (65) vorgesehen ist, dessen Durchmesser in einem Ausgangszustand vor und während der Einführung höchstens gleich dem Durchmesser des Grundkörpers und in einem aufgeweiteten Endzustand größer als dieser ist.

6. Gefäßelektrodenleitung nach Anspruch 4 oder 5, **dadurch gekennzeichnet, daß** mindestens ein Teil der Oberfläche der elastischen Wendel (55) oder des rohrförmigen Elements (65) nichtleitend ausgeführt ist.

7. Gefäßelektrodenleitung nach einem der Ansprüche 1 bis 6, **dadurch gekennzeichnet, daß** das Fixierungsmittel (35; 45; 55; 65; 75) flexibel ausgebildet ist.

8. Gefäßelektrodenleitung nach einem der vorhergehenden Ansprüche, **dadurch gekennzeichnet, daß** die Elektrode oder die distalste von mehreren Elektroden (13; 33; 43) einen Abstand zwischen 30 und 80 mm vom distalen Ende des Grundkörpers (11; 21; 41) hat.

9. Gefäßelektrodenleitung nach einem der vorhergehenden Ansprüche, **gekennzeichnet durch** zwei in einem Abstand zwischen 25 und 80 mm voneinander angeordnete Elektroden (13, 14; 23, 24; 33, 34; 43, 44).

10. Gefäßelektrodenleitung nach einem der vorhergehenden Ansprüche, **dadurch gekennzeichnet, daß** die Elektrode oder Elektroden (13, 14; 23, 24; 33, 34; 43, 44) eine Länge zwischen 2 und 3 mm und eine geometrische Oberfläche zwischen 10 und 25 mm² haben.

11. Gefäßelektrodenleitung nach einem der vorhergehenden Ansprüche, **gekennzeichnet durch** eine das Einwachsen im umgebendes Gewebe positiv beeinflussende Wirkstoffbeschichtung, insbesondere Kollagen- oder Fibronectin-Beschichtung.

## Claims

1. Electrode cable (50, 60, 70) for attachment to a vessel wall, for stimulating a pacemaker and/or for sensing cardiac functions in the region of the atrium, comprising an extended base body (51, 61, 71) receiving at least one electrode cable, and at least one electrode (53, 54, 63, 64, 73, 74) attached to the base body, the effective diameter of the electrode or electrodes (53, 54, 63, 64, 73, 74) being approximately equal to the diameter of the base body (51, 61, 71), and fixing means (55, 65, 75), which do not substantially act as an electrode and which, in particular, at least in part, comprise an insulating surface, for fixedly positioning the electrode or electrodes (53, 54, 63, 64, 73, 74) with respect to the vessel wall, being present, **characterised in that** a fixing means (55, 65, 75), which is attached to a fixing means carrier body (56, 66, 76) that is axially displaceable in relation to the base body (51, 61, 71) and may, in its axial position, be connected thereto, is provided in the distal end region of the base body (51, 61, 71) for fixing in the coronary sinus or in a side vessel thereof, having a lateral extension adjusted to the diameter of the vessel, in particular 3 to 6 mm, and the base body or the fixing means carrier body (56, 66, 76) being highly flexible in this region.

2. Electrode cable for attachment to a vessel wall according to claim 1, **characterised in that** a plastics material worm thread portion (35; 75) surrounding the distal end of the fixing means carrier body (71) and made, in particular, from the material of the base body (31) or the fixing means carrier body (71), and comprising one to three turns, is provided as the fixing means.

3. Electrode cable for attachment to a vessel wall according to claim 1, **characterised in that** a large number of plastics materials fins (45), which are resiliently inclined toward the proximal end, relative to the longitudinal axis of the base body (41), is provided as the fixing means.

4. Electrode cable for attachment to a vessel wall according to claim 1, **characterised in that** a resilient helix (55), the diameter of which during insertion, in a state extended by an insertion-assisting means (58), is at most equal to the diameter of the base body, and in the relaxed state is less than said diameter, is provided as the fixing means.

5. Electrode cable for attachment to a vessel wall according to claim 1, **characterised in that** an expandable tubular element (65), the diameter of which in an initial state, before and during insertion, is at most equal to the diameter of the base body, and in an expanded final state is larger than said diameter, is provided as the fixing means.

6. Electrode cable for attachment to a vessel wall according to either claim 4 or claim 5, **characterised in that** at least a portion of the surface of the resilient helix (55) or the tubular element(65) is configured in a non-conductive manner.

7. Electrode cable for attachment to a vessel wall according to any one of claims 1 to 6, **characterised in that** the fixing means (35; 45; 55; 65; 75) is flexible in its configuration.

8. Electrode cable for attachment to a vessel wall according to any one of the preceding claims, **characterised in that** the electrode or the most distal of a plurality of electrodes (13; 33; 43) is at a distance of between 30 and 80 mm from the distal end of the base body (11; 21; 41).

9. Electrode cable for attachment to a vessel wall according to any one of the preceding claims, **characterised by** two electrodes (13, 14; 23, 24; 33, 34; 43, 44) disposed at a distance of between 25 and 80 mm from each other.

10. Electrode cable for attachment to a vessel wall according to any one of the preceding claims, **characterised in that** the electrode or electrodes (13, 14; 23, 24; 33, 34; 43, 44) has/have a length of between 2 and 3 mm and a geometric surface of between 10 and 25 mm².

11. Electrode cable for attachment to a vessel wall according to any one of the preceding claims, **characterised by** an active ingredient coating, in particular a collagen or fibronectin coating, positively influencing incorporation in the surrounding tissue.

## Revendications

1. Ligne à électrodes (50, 60, 70) pour vaisseau, pour la stimulation d'un stimulateur et/ou pour la détection d'actions cardiaques au niveau de l'oreillette, comportant un corps de base allongé (51,61,71), qui loge au moins une ligne à électrodes, et au moins une électrode (53,54,63,64,73,74) montée sur le corps de base, dans laquelle le diamètre effectif de la ou des électrodes (53, 54,63,64,73,74) est approximativement égal au diamètre du corps de base (51,61,71), et des moyens de fixation (55,65, 75), qui pour l'essentiel n'agissent pas en tant qu'électrode et comportent notamment au moins en partie une surface isolante et sont prévus pour positionner de façon fixe la ou les électrodes (53,54,63,64,73,74) par rapport à la paroi du vaisseau, **caractérisée en ce que** dans la partie d'extrémité du corps de base (51,61,71) il est prévu un moyen de fixation (55,65,75) qui est monté sur un corps de support (56,66,76) du moyen de fixation, qui est déplaçable axialement par rapport au corps de base (51,61,71) et peut être fixé dans sa position axiale sur ce dernier, le moyen de fixation étant destiné à être fixé dans le sinus coronarien ou dans un vaisseau proche de ce dernier, avec une étendue latérale déterminée accordée sur le diamètre du vaisseau, notamment 3 à 6 mm, et que le corps de base ou le corps de support (56,66,76) du moyen de fixation est flexible à un degré important dans cette zone.

2. Ligne à électrodes pour vaisseau selon la revendication 1, **caractérisée en ce qu'**il est prévu comme moyen de fixation une section filetée de vis en matière plastique (35;75), qui entoure l'extrémité distale du corps de support (71) du moyen de fixation et est réalisée notamment avec le matériau du corps de base (31) ou du corps de support (71) du moyen de fixation et comportant une à trois spires.

3. Ligne à électrodes pour vaisseau selon la revendication 1, **caractérisée en ce qu'**il est prévu comme moyen de fixation une multiplicité de pennes en matière plastique (45) qui sont inclinées élastiquement par rapport à l'axe longitudinal du corps de base (41) en direction de l'extrémité proximale.

4. Ligne à électrodes pour vaisseau selon la revendication 1, **caractérisée en ce qu'**il est prévu comme moyen de fixation un filament hélicoïdal élastique (55), dont le diamètre pendant l'introduction, dans un état étiré par un moyen auxiliaire d'entrée (58), est égal au maximum au diamètre du corps de base, et à l'état détendu, est supérieur à ce diamètre.

5. Ligne à électrodes pour vaisseau selon la revendication 1, **caractérisée en ce qu'**il est prévu comme moyen de fixation un élément de forme tubulaire pouvant élargi (65), dont le diamètre, dans un état initial avant et pendant l'introduction, est égal au maximum au diamètre du corps de base et, dans un état final élargi, est supérieur à ce diamètre.

6. Ligne à électrodes pour vaisseau selon la revendication 4 ou 5, **caractérisée en ce qu'**au moins une partie de la surface du filament hélicoïdal élastique (55) ou de l'élément de forme tubulaire (65) est agencée de manière à être non conductrice.

7. Ligne à électrodes pour vaisseau selon l'une des revendications 1 à 6, **caractérisée en ce que** le moyen de fixation (35;45;55;65;75) est agencé de manière à être flexible.

8. Ligne à électrodes pour vaisseau selon l'une des revendications précédentes, **caractérisée en ce que** l'électrode ou l'électrode la plus distale parmi plusieurs électrodes (13;33;43) est séparée de l'extrémité distale du corps de base (11;21;41) par une distance comprise entre 30 et 80 mm.

9. Ligne à électrodes pour vaisseau selon l'une des revendications précédentes, **caractérisée par** deux électrodes (13,14;23,24;33,34;43,44) disposées à une distance réciproque comprise entre 25 et 80 mm.

10. Ligne à électrodes pour vaisseau selon l'une des revendications précédentes, **caractérisée en ce que** la ou les électrodes (13,14;23,24;33,34;43,44) possèdent une longueur comprise entre 2 et 3 mm et une surface géométrique comprise entre 10 et 25 mm².

11. Ligne à électrodes pour vaisseau selon l'une des revendications précédentes, **caractérisée par** un revêtement de matière active, qui a une influence positive sur la croissance du tissu environnant, et notamment un revêtement de collagène ou de fibronectine.
